# EUROPEAN PATENT APPLICATION

(11) **EP 4 825 376 A1**
(43) Date of publication of application: **21.10.2026**
(21) Application number: 24903017.2
(22) Date of filing: 23.12.2024
(51) Int. Cl.: A61K 9/52, A61K 31/352, A61K 47/42, A61P 29/00, A61P 3/00

(54) **MOLECULAR CAPSULE COMPRISING QUERCETIN COATED WITH POLYPEPTIDES AND PREPARATION METHOD THEREFOR**

(30) Priority: 13.12.2023 CN 202311714300
(71) Applicant: HUIHEGU (SHANGHAI) BIOTECHNOLOGY CO., LTD., Pudong New Area, Shanghai 200120 (CN)
(72) Inventor: MEI, Hexiang, Shanghai 200120 (CN); REN, Chuanpeng, Shanghai 200120 (CN); GAO, Ying, Shanghai 200120 (CN); WU, Di., Shanghai 200120 (CN); ZHOU, Mengya, Shanghai 200120 (CN)
(74) Representative: Izquierdo Blanco, Maria Alicia
(86) International application number: PCT/CN2024/141308
(87) International publication number: WO 2025/124602

(57) **Abstract**

The present disclosure belongs to the technical field of molecules, and particularly relates to a polypeptide-encapsulated quercetin molecular capsule and a preparation method therefor. The method includes steps of: (1) mixing amino acid A, quercetin, and water in a ratio, followed by ultrasonic enzymolysis to prepare a precursor; (2) mixing the precursor with amino acid B in a ratio to prepare a molecular capsule precursor; and (3) adding the molecular capsule precursor into a crystallizer for recrystallization of an outer structure, and performing filtering and high-pressure drying to prepare the polypeptide-encapsulated quercetin molecular capsule. The present disclosure can effectively improve the bioavailability of the quercetin in the constructed molecular capsule by optimizing the powder properties of the drug crystal, and implement controllable sustained release through a specific particle size, thus better controlling the release rate and release amount of the quercetin.

## Description

### TECHNICAL FIELD

The present disclosure belongs to the technical field of molecules, and particularly relates to a polypeptide-encapsulated quercetin molecular capsule and a preparation method therefor.

### BACKGROUND

Quercetin, also known as Meletin, Quercetine, and Quercetol, is a plant flavonol belonging to a flavonoids compound in polyphenols, and has been found in plants such as fruits, vegetables, and cereals. At present, no definitive research has confirmed that the quercetin has therapeutic effects on diseases. However, the study by C L T J H A B, A L G P L P, C S A B, et al. (Senolytics decrease senescent cells in humans: Preliminary report from a clinical trial of Dasatinib plus Quercetin in individuals with diabetic kidney disease. [J].) has demonstrated the potential value and efficacy of the quercetin in clinical medicine. This has further drawn attention to the research and application of the quercetin.

In recent years, the study by Stewart L K, Soileau J L, Ribnicky D, et al. (Quercetin transiently increases energy expenditure but persistently decreases circulating markers of inflammation in C57BL/6J mice fed a high-fat diet. [J].) and some other researches have also indicated that the quercetin has great application prospects for topical use, and can achieve anti-inflammatory and metabolism-promoting effects.

Nevertheless, the current research has revealed major limitations in the application of the quercetin. Specifically, its high release rate and low release depth result in low actual effective utilization.

In view of this, the mainstream research direction involves the construction of container molecules for encapsulating the quercetin to form molecular capsules. Particularly notable are reversible supramolecular capsules constructed through non-covalent interactions, such as hydrogen bonding, electrostatic interactions, halogen bonding, hydrophobic interactions, and metal coordination bonding. However, existing technical solutions for preparing molecular capsules encapsulating the quercetin have some technical deficiencies, such as low drug loading capacity, poor bioavailability, strong drug irritation, and excessively slow sustained release of drugs.

### SUMMARY

To overcome existing application defects of quercetin and solve problems such as low drug loading capacity, poor bioavailability, strong irritation, and poor release controllability of existing quercetin molecular capsules, the present disclosure provides a polypeptide-encapsulated quercetin molecular capsule and a preparation method therefor.

Main objectives of the present disclosure are as follows:
1. To improve the biosafety of the quercetin molecular capsule and reduce its irritation;
2. To increase the drug loading capacity of the quercetin molecular capsule; and
3. To implement controllable release so as to enhance actual utilization effects of the quercetin molecular capsule.

In order to achieve the above objectives, the present disclosure adopts the following technical solution.

According to a preparation method for a polypeptide-encapsulated quercetin molecular capsule, the method includes steps of:
(1) mixing amino acid A, quercetin, and water in a ratio, followed by ultrasonic enzymolysis to prepare a precursor;
(2) mixing the precursor with amino acid B in a ratio to prepare a molecular capsule precursor; and
(3) adding the molecular capsule precursor into a crystallizer for recrystallization of an outer structure, and performing filtering and high-pressure drying to prepare the polypeptide-encapsulated quercetin molecular capsule.

Preferably,
the amino acid A in the step (1) is L-histidine; and
the amino acid A, the quercetin, and the water are mixed in a mass ratio of (0.9 to 1.2):1:(3 to 5) in the step (1).

Preferably,
the ultrasonic enzymolysis in the step (1) is performed using a neutral protease having an activity of 2,000 U/g to 3,000 U/g on a dry basis; and
the neutral protease is used in an amount of 8 wt% to 12 wt% based on the quercetin.

Preferably,
the amino acid B in the step (2) is a mixture of D-β-alanine and L-histidine; and
in a total amount of the amino acid B, a content of the L-histidine ranges from 28 wt% to 32 wt%, with the balance being the D-β-alanine.

Preferably,
during the preparation of a molecular capsule precursor in the step (2), a reaction temperature is controlled at a range from 45°C to 50°C, a pH value of a reaction system is adjusted at a range from 6.8 to 7.2, and in a reaction process, liquid proteinase K having an activity of 600 U/mL is added in an amount from 4 wt% to 6 wt% based on the amino acid B.

Preferably,
the recrystallization in the step (3) is performed at a temperature of 30°C to 35°C, a rotational speed of the crystallizer is controlled at a range from 300 rpm to 400 rpm during the recrystallization, and the recrystallization lasts for 1 h to 2 h.

Preferably,
the high-pressure drying is performed at a temperature of 24°C to 26°C and a pressure of 4 MPa to 6 MPa for 4 h to 5 h.

A polypeptide-encapsulated quercetin molecular capsule is also disclosed.

The quercetin directly reacts with superoxide anions and hydroxyl free radicals through single-electron transfer of an o-dihydroxyphenyl group, acting as a hydrogen donor. In this process, the quercetin forms more stable intramolecular hydrogen bonds, preventing the peroxidation of the unsaturated fatty acid and arachidonic acid and reducing damage to biological membranes. The quercetin can act on free radical-related enzymes to cause protein precipitation and prevent synthesis of oxygen free radicals. Further, the quercetin can chelate metal ions in cells to inhibit the generation of the hydroxyl free radicals.

A nano drug delivery system is a special dosage form that uses nanoparticles to load drugs (in the present disclosure, unless otherwise specified, the term "drug" specifically refers to the quercetin), which can solve various problems existing in drug application. A biological membrane-based nano drug delivery system, as a biomimetic drug carrier, exhibits superior performance.

A biological membrane has an outer layer composed of a phospholipid bilayer and an internal cavity capable of loading macromolecules, small molecules, and nucleic acid substances. The biological membrane has desirable biotransportability and sustained-release effects. However, during use, problems of compatibility between the biological membrane and the drug and instability of the drug itself result in low drug encapsulation and loading efficiency, uneven encapsulation and loading, and poor stability.

In response, the present disclosure uses L-carnosine as a base material to prepare the molecular capsule through an entrapment and complexation method. As a bioactive polypeptide, the L-carnosine has broad application prospects in medicine, cosmetics, health products, food, etc. Meanwhile, the molecular capsule technology can isolate a core material from a surrounding environment, thus reducing damage to the core material caused by the external environment (such as water, light, oxygen, and temperature). In this way, the appearance and properties of the core material are improved, and storage, transportation, and consumption of the nutrients are facilitated. Furthermore, a capsule wall membrane has a sustained-release function, which can control the release speed of the core material having a biologically active ingredient to enable optimal efficacy in a human body. Moreover, a wall material exerts a shielding effect, concealing undesirable colors, odors, bitter taste, etc., of the core material. In view of this, the technology is widely used in food, medicine, biology, and other fields.

In the technical solution of the present disclosure, the molecular capsule is a special structure having a unique three-dimensional cavity, formed by two or more molecules linked together through reversible intermolecular non-covalent interactions. Such a structure has potential application values in the fields of drug release control, catalysis, separation, materials science, and biomedicine. The present disclosure further employs ultrasonic enzymolysis to treat amino acid and quercetin molecules, accelerating the unfolding of amino acid molecules. In this way, the hydrolysis rate is improved. The ultrasonic enzymolysis can not only improve the solubility of a precursor and enhance the activity of angiotensin-converting enzyme (ACE) inhibitory peptides, but also produce a large number of precursors having higher antioxidant activity. The ultrasonically treated precursors can be directly used to prepare hypoallergenic bioactive peptides.

In the construction process, the carnosine itself exhibits dual-end binding characteristics with the quercetin. The carnosine is a dipeptide composed of the L-histidine and the D-β-alanine as monomers, and both of them can form intermolecular bonds with the quercetin. Thus, if the carnosine is directly used to be bound with the quercetin, different volume effects of the two monomers lead to a disordered actual binding mode of the carnosine, which ultimately reduces the drug loading capacity to a certain extent. In addition, the uneven distribution of extrinsic components and active groups results in weakened permeability, as well as reduced drug loading capacity and poor controllability of drug release during practical application. However, in the present disclosure, the monomer L-histidine is first used to implement preliminary binding with the quercetin to form the precursor, and then the D-β-alanine is added to complete reaction construction of the carnosine. In this way, a binding mode between the carnosine and the quercetin can be effectively ensured, thus ensuring the stability of final product properties and effects.

In the formed molecular capsule, on one hand, the wall material of the molecular capsule plays a decisive role in drug effects, affecting the fluidity, permeability, solubility, and sustained-release performance of the encapsulated product. An ideal wall material needs to be compatible with the core material, have desirable stability, hygroscopicity, and solubility, maintain stable chemical properties, and be derived from widely available, low-cost sources. On the other hand, the molecular capsule with the carnosine as a base material has a framework with a cavity and bonding units capable of reversible interactions. Due to the structural characteristics of a wider upper part and a narrower lower part with an internal "cup-shaped" cavity, carnosine molecules can easily be derivatized to introduce specific functional groups, making them an ideal framework for constructing a supramolecular capsule.

In the technical solution of the present disclosure, recrystallization of the outer structure is performed. After modification, the obtained product has a spherical shape, with aggregation traces being not disordered. The traces form a polycrystalline aggregate with a radially ordered arrangement. Initially, the aggregate forms filamentous fibers, and then starts to branch and grow from two ends of the fibers to form "sheaf-shaped" crystals having a narrower middle part and two thicker ends. Finally, through continuous small-angle branching, spherical spherocrystals are formed. Through spherical crystallization technology, small-sized acicular quercetin is converted into compact spherical crystals. The conversion process improves the sphericity and particle size of the product, and meanwhile, reduces the electrostatic interactions and frictional forces between crystals. Therefore, the method significantly improves the powder properties of the active pharmaceutical ingredient, such as fluidity, compressibility, and bulk densities. Through the spherical crystallization technology, spherical microparticles with the quercetin encapsulated using the carnosine as a carrier are prepared, such that the sustained and controlled release of the quercetin can be achieved.

In the technical solution of the present disclosure, researchers successfully prepare a spherocrystal with specific properties. The entropy value of the spherocrystal is significantly low, such that its highly ordered structure can be maintained, and its shape and size can be better kept. In addition, the melting point of the spherocrystal is extremely high, indicating that the spherocrystal can maintain stable performance in a high-temperature environment and exhibit excellent thermal stability. Both the solubility and dissolution rate of the spherocrystal are remarkably low, allowing it to be released at a slow rate in vivo, such that the drug efficacy of the spherocrystal can be better exerted. Such a low release rate not only improves the bioavailability of the drug, but also effectively reduces side effects, making the drug safer and more effective. To summarize, with numerous advantages such as low entropy value, high melting point, low solubility and dissolution rate, and high bioavailability, the spherocrystal prepared by the technical solution of the present disclosure becomes an ideal form of a biopharmaceutical preparation and has broad application prospects.

The present disclosure has the following beneficial effects:
The present disclosure can effectively improve the bioavailability of the quercetin in the constructed molecular capsule by optimizing the powder properties of the drug crystal, and implement controllable sustained release through a specific particle size, thus better controlling the release rate and release amount of the quercetin.

### BRIEF DESCRIPTION OF THE DRAWINGS

FIG. 1 shows skin permeation of a moisturizing lotion;
FIG. 2 shows skin permeation of a repair eye cream;
FIG. 3 shows skin permeation condition of a serum;
FIG. 4 shows drug uptake by cells in a moisturizing lotion control group after 4 h;
FIG. 5 shows drug uptake by cells in a moisturizing lotion test group after 4 h;
FIG. 6 shows drug uptake by cells in a repair eye cream control group after 4 h;
FIG. 7 shows drug uptake by cells in a repair eye cream test group after 4 h;
FIG. 8 shows drug uptake by cells in a serum control group after 4 h; and
FIG. 9 shows drug uptake by cells in a serum test group after 4 h.

### DETAILED DESCRIPTION

The present disclosure will be further described in detail with reference to specific examples and drawings in the description. Those of ordinary skill in the art can implement the present disclosure based on the description. In addition, examples of the present disclosure involved in the following description are generally merely some rather than all examples of the present disclosure. In view of this, based on the examples of the present disclosure, all other examples obtained by those of ordinary skill in the art without making inventive efforts should fall within the scope of protection of the present disclosure.

Unless otherwise specified, raw materials used in the examples of the present disclosure are all commercially available or obtainable by those skilled in the art. Unless otherwise specified, the methods used in the examples of the present disclosure are all within the knowledge of those skilled in the art.

### Example 1

According to a preparation method for a polypeptide-encapsulated quercetin molecular capsule, including following steps:
(1) L-histidine, quercetin, and distilled water were uniformly mixed in a mass ratio of 0.9:1:3, and subjected to ultrasonic enzymolysis using 8 wt% of a neutral protease (2,000 U/g, dry basis) based on a mass of the quercetin, at a temperature of 43°C, a pH value of 6.8, and an ultrasonic frequency of 170 W for 3 h to prepare a precursor;
(2) the precursor was uniformly mixed with amino acid B in a mass ratio of 1:0.8, and reaction was performed at a temperature of 45°C and a pH value of 6.8 for 3 h to prepare a molecular capsule precursor, during which 4 wt% of proteinase K (600 U/mL), based on a mass of the amino acid B, was also added, where the amino acid B was a mixture of D-β-alanine and L-histidine, with the L-histidine accounting for 28 wt% and the balance being the D-β-alanine; and
(3) the molecular capsule precursor was added into a crystallizer, recrystallization of an outer structure was performed at a temperature of 30°C and a rotational speed of 300 rpm for 1 h, filtering was performed to retain a solid phase, then drying was performed at a temperature of 24°C and a pressure of 6 MPa for 4 h, and the polypeptide-encapsulated quercetin molecular capsule was prepared.

The following performance tests were performed on the prepared polypeptide-encapsulated quercetin molecular capsule, and specific characterization results were as follows.

Antioxidant activity assay:
Superoxide anion (O²⁻) free radical scavenging rate: A Tris-HCl solution (0.05 mol/L, pH 8.2) was prepared using 0.1 mol/L of HCl, 4.5 mL of the Tris-HCl solution was added into 4.2 mL of a molecular capsule aqueous solution, and after uniform mixing, the mixture was incubated in a thermostatic water bath at 25°C for reaction for 20 min. 3 mmol/L of pyrogallol was pre-heated in a thermostatic water bath at 25°C. After 0.3 mL of pyrogallol was added into the heated reaction liquid, absorbance at 300 nm was immediately measured, the absorbance was measured every 30 s, and the superoxide anion free radical scavenging rate was calculated based on the slope of the absorbance within 4 min. A specific formula was as follows: ${CL}_{{O}^{2 -}} = \frac{{K}_{0} - K}{K} \times 100 %$

In the formula:
K₀: control value obtained with distilled water; and
K: value obtained with the sample.

Reducing power assay: A phosphate buffer solution (0.2 mol/L, pH 6.6) was prepared, and 2.5 mL of the phosphate buffer solution and 2.5 mL of potassium ferricyanide (1%) were added into 1 mL of a molecular capsule to-be-tested solution. After uniform mixing, the mixture was incubated in a thermostatic water bath at 50°C for reaction for 20 min, and then 2.5 mL of trichloroacetic acid (10%) was added to terminate the reaction. After centrifugation at 5,000 rpm for 10 min, 2.5 **mL** of supernatant was collected and mixed with 2.5 **mL** of distilled water and 0.5 **mL** of FeCl₃, and still standing was performed for 10 min. Absorbance was measured at 300 nm. The absorbance value of the sample represented its reducing power.

Hydroxyl free radical (OH·) scavenging activity assay: Test samples a, b, and c were prepared according to the following characteristics. 1 **mL** of each test sample, 6 mmol/L ferrous sulfate, and a 6 mmol/L salicylic acid-ethanol solution were collected and mixed uniformly. Then, 1 mL of saturated H₂O₂ was added to initiate reaction. The mixture was incubated in a thermostatic water bath at 37°C for 30 min, then absorbance of the reaction liquid was measured at 510 nm, and the hydroxyl free radical (OH·) scavenging rate was calculated. A specific formula was as follows: ${CL}_{\left(OH⋅\right)} = \left(1-\frac{{V}_{a} - {V}_{b}}{{V}_{c}}\right) \times 100 %$

| | Molecular capsule aqueous solution (mL) | Ferrous sulfate (mL) | Salicylic acid-ethanol solution (mL) | Ethanol (mL) | Distilled water (mL) | Saturated H2O2 (mL) |
|---|---|---|---|---|---|---|
| a | 1 | 1 | 1 | - | - | 1 |
| b | 1 | 1 | - | 1 | - | 1 |
| c | - | 1 | 1 | - | 1 | 1 |

Angiotensin-converting enzyme (ACE) inhibition rate assay: A 0.1 mol/L phosphate buffer solution was prepared through 0.3 mol/L sodium chloride, and the pH of the buffer solution was adjusted to 8.3. Then, the buffer solution was used to prepare a 5 mmol/L hippuryl-histidyl-leucine (HHL) solution, and the addition amounts of all to-be-tested reagents were characterized as follows. After reaction, 0.4 mL of ethyl acetate was added to extract hippuric acid from the reaction liquid, centrifugation was performed at 4,000 rpm for 10 min after uniform shaking, 0.3 mL of supernatant was collected and put into a clean centrifuge tube, and liquid in the centrifuge tube was heated and dried. The liquid was cooled to a room temperature, and then 0.6 mL of ultrapure water was added to extract the sample attached to the tube wall. Absorbance was measured at 228 nm, and the ACE inhibition rate was calculated. A specific formula was as follows: $ACE inhibition rate = \frac{\left({A}_{II}-{A}_{I}\right)}{\left({A}_{II}-{A}_{III}\right)} \times 100 %$

In the formula:
A_{I}: sample group;
A_{II}: control group; and
A_{III}: early termination reaction group.

| | Molecular capsule aqueous solution (mL) | Saturated hydrochloric acid (mL) | HHL (mL) | ACE (mL) | Distilled water (mL) |
|---|---|---|---|---|---|
| I | 5 | 0 | 15 | 0 | 0 |
| II | 0 | 0 | 15 | 0 | 5 |
| III | 5 | 50 | 15 | 5 | 0 |

The test results were shown in the following table:

| Superoxide anion (O²⁻) free radical scavenging rate, % | Reducing power | Hydroxyl free radical (OH·) scavenging rate, % | ACE inhibition rate, % |
|---|---|---|---|
| 56.6 | 0.39 | 67.8 | 65.2 |

In addition to the above efficacy assays, the polypeptide-encapsulated quercetin molecular capsule prepared in this example was further subjected to an in vitro release test, a transdermal release test, and an in vitro permeation test.

The in vitro release test was performed using a RYJ-12 transdermal diffusion tester. A control group and a test group were set up, with three parallel tests for each group. The control group consisted of an unencapsulated mixture of quercetin and carnosine (in a mass ratio of quercetin to carnosine of 1:1.2). The test group was the carnosine-encapsulated quercetin molecular capsule prepared in this example. Both the diffusion cell and the receiver cell used a phosphate buffered saline (PBS)-absolute ethyl alcohol mixed solution in a volume ratio of 4:1. For the diffusion cell, propylene glycol was used to dilute the control group and the test group to a concentration of 2 mg/mL. In this test, an artificial semipermeable membrane was used to simulate the in vitro release platform. The artificial semipermeable membrane was placed in a transdermal tester to withdraw 1 mL of the solution in the receiver cell at intervals of 1 h, and then an isotonic solution of a corresponding volume was added. After sampling at all time points was completed, the solution was placed in an ultraviolet spectrophotometer to measure the corresponding absorbance, and the in vitro release rate was calculated according to a quercetin-carnosine standard curve.

| Time, h | 0 | 4 | 8 | 12 | 16 | 20 | 24 | 28 | 32 | 36 |
|---|---|---|---|---|---|---|---|---|---|---|
| Control group, % | 0 | 23 | 32 | 45 | 59 | 71 | 80 | 83 | 84 | 86 |
| Test group, % | 0 | 11 | 23 | 33 | 42 | 50 | 56 | 60 | 63 | 65 |

The transdermal release test was conducted under the following conditions: temperature of 37°C; a medium consisting of 80% isotonic phosphate buffer solution (pH 7.4) mixed with 20% absolute ethyl alcohol; and a RYJ-12 transdermal diffusion tester. A test group and a control group were set up, with three parallel tests for each group. The control group consisted of an unencapsulated mixture of quercetin and carnosine (in a mass ratio of quercetin to carnosine of 1:1.2). The test group was the carnosine-encapsulated quercetin molecular capsule prepared in this example. The depilated abdominal skin of a 6-week-old Wistar rat was used. After respiratory anesthesia, the rat was depilated, killed, and dissected. The skin of the rat was excised, the fat layer was removed, and the skin was washed thoroughly with clean water, cut into appropriately sized pieces, and placed in normal saline at 4°C for later use. The drug used in the diffusion cell was diluted with propylene glycol, with a concentration of 2 mg/mL and an addition amount of 1 mL. The receiver cell contained 80% isotonic phosphate buffer solution (pH 7.4) mixed with 20% absolute ethyl alcohol. The skin was placed between the two cells, and the assembly was clamped and placed in the transdermal tester. At corresponding intervals, 1 mL of the solution in the receiver cell was withdrawn, and then the corresponding volume of medium solution was added. After sampling at all time points was completed, the solution was placed in an ultraviolet spectrophotometer to measure absorbance, the concentration and the transmittance were calculated according to a standard curve of the quercetin, and a chart was drawn.

| Time, h | 0 | 4 | 8 | 12 | 16 | 20 | 24 | 28 | 32 | 36 |
|---|---|---|---|---|---|---|---|---|---|---|
| Control group, % | 0 | 21 | 38 | 43 | 49 | 53 | 55 | 56 | 56 | 58 |
| Test group, % | 0 | 29 | 53 | 69 | 77 | 83 | 86 | 89 | 91 | 92 |

For the in vitro permeation test, fresh abdominal skin of a three-month-old miniature pig was used. After depilation, the fat layer and part of the dermis were scraped off using a scraper. The skin was cut into circular pieces with a size of about 3.14 cm² and fixed to a permeation device with the stratum corneum facing upward. Fluorescein isothiocyanate-hyaluronic acid (FITC-HA) was used as the fluorescein in the test. After the quercetin was crosslinked with the fluorescein, the drug was loaded by the encapsulation technology in this example. The drug was diluted to a concentration of 50 µg/mL (based on the FITC-HA) through a PBS buffer solution (pH 7.4) for both the test group and the control group. The control group consisted of an unencapsulated mixture of quercetin and carnosine (in a mass ratio of quercetin to carnosine of 1:1.2). The test group was the carnosine-encapsulated quercetin molecular capsule prepared in this example. 0.5 mL of the diluted solution was added to the permeation device and stored in the dark for 30 min. Subsequently, the skin was taken off and washed thoroughly with clean water until fluorescence on the surface layer was not discolored. The surface water was withdrawn with absorbent paper, the skin was placed in the dark for 10 min to slightly air-dry, and then the skin was sliced and placed under a fluorescence microscope for observation of fluorescence permeation in the skin.

| | | | |
|---|---|---|---|
| Control group permeation depth | 239±56 µm | Test group permeation depth (µm) | 552±21 µm |

From the above in vitro characterization results, it can be clearly seen that the molecular capsule of the present disclosure had obvious sustained-release characteristics in vitro, and can maintain relatively stable and slow release within 36 h. In contrast, the sample of the control group was released quickly and intensively within about the first 24 h, indicating its poor release controllability. And the polypeptide-encapsulated quercetin molecular capsule of the present disclosure can be used for long-term release components in vitro through stable and sustained-release characteristics, so as to stably provide the quercetin. In addition, the molecular capsule of the present disclosure exhibited an extremely high transdermal release rate and a significantly high release depth. This was primarily attributable to the polypeptide shell layer, enabling excellent skin permeability of the entire molecular capsule. Thus, when used in skin care products and common cosmetic additives, the molecular capsule of the present disclosure can achieve stable release and rapid penetration and absorption after release, such that the anti-inflammatory ability of the quercetin itself can be effectively exerted.

### Example 2

According to a preparation method for a polypeptide-encapsulated quercetin molecular capsule, including following steps:
(1) L-histidine, quercetin, and distilled water were uniformly mixed in a mass ratio of 1:1:4, and subjected to ultrasonic enzymolysis using 10 wt% of a neutral protease (2,500 U/g, dry basis) based on a mass of the quercetin, at a temperature of 45°C, a pH value of 7, and an ultrasonic frequency of 150 W for 2.5 h to prepare a precursor;
(2) the precursor was uniformly mixed with amino acid B in a mass ratio of 1:1, and reaction was performed at a temperature of 47°C and a pH value of 7 for 2.5 h to prepare a molecular capsule precursor, during which 5 wt% of proteinase K (600 U/mL), based on a mass of the amino acid B, was also added, where the amino acid B was a mixture of D-β-alanine and L-histidine, with the L-histidine accounting for 30 wt% and the balance being the D-β-alanine; and
(3) the molecular capsule precursor was added into a crystallizer, recrystallization of an outer structure was performed at a temperature of 33°C and a rotational speed of 350 rpm for 1.5 h, filtering was performed to retain a solid phase, then drying was performed at a temperature of 25°C and a pressure of 5 MPa for 4.5 h, and the polypeptide-encapsulated quercetin molecular capsule was prepared.

The molecular capsule prepared in this example was subjected to an in vitro release test, a transdermal release test, and an in vitro permeation test that are the same as those in Example 1.

Test results were shown in the following table:

| In vitro release test | | | | | | | | | | |
|---|---|---|---|---|---|---|---|---|---|---|
| Time, h | 0 | 4 | 8 | 12 | 16 | 20 | 24 | 28 | 32 | 36 |
| Control group, % | 0 | 23 | 32 | 45 | 59 | 71 | 80 | 83 | 84 | 86 |
| Test group, % | 0 | 11 | 23 | 33 | 41 | 50 | 57 | 58 | 62 | 63 |

| Transdermal release test | | | | | | | | | | |
|---|---|---|---|---|---|---|---|---|---|---|
| Time, h | 0 | 4 | 8 | 12 | 16 | 20 | 24 | 28 | 32 | 36 |
| Control group, % | 0 | 21 | 38 | 43 | 49 | 53 | 55 | 56 | 56 | 58 |
| Test group, % | 0 | 28 | 51 | 69 | 74 | 83 | 88 | 90 | 93 | 97 |

| In vitro permeation test | | | | | | | | | | |
|---|---|---|---|---|---|---|---|---|---|---|
| Control group permeation depth | 239±56 µm | | | | Test group permeation depth (µm) | | | 549±25 µm | | |

### Example 3

According to a preparation method for a polypeptide-encapsulated quercetin molecular capsule, including following steps:
(1) L-histidine, quercetin, and distilled water were uniformly mixed in a mass ratio of 1.2:1:5, and subjected to ultrasonic enzymolysis using 12 wt% of a neutral protease (3,000 U/g, dry basis) based on a mass of the quercetin, at a temperature of 47°C, a pH value of 7.2, and an ultrasonic frequency of 130 W for 2 h to prepare a precursor;
(2) the precursor was uniformly mixed with amino acid B in a mass ratio of 1:1.2, and reaction was performed at a temperature of 50°C and a pH value of 7.2 for 2 h to prepare a molecular capsule precursor, during which 6 wt% of proteinase K (600 U/mL), based on a mass of the amino acid B, was also added, where the amino acid B was a mixture of D-β-alanine and L-histidine, with the L-histidine accounting for 32 wt% and the balance being the D-β-alanine; and
(3) the molecular capsule precursor was added into a crystallizer, recrystallization of an outer structure was performed at a temperature of 35°C and a rotational speed of 400 rpm for 1 h, filtering was performed to retain a solid phase, then drying was performed at a temperature of 26°C and a pressure of 4 MPa for 5 h, and the polypeptide-encapsulated quercetin molecular capsule was prepared.

The molecular capsule prepared in this example was subjected to an in vitro release test, a transdermal release test, and an in vitro permeation test that are the same as those in Example 1.

Test results were shown in the following table:

| In vitro release test | | | | | | | | | | |
|---|---|---|---|---|---|---|---|---|---|---|
| Time, h | 0 | 4 | 8 | 12 | 16 | 20 | 24 | 28 | 32 | 36 |
| Control group, % | 0 | 23 | 32 | 45 | 59 | 71 | 80 | 83 | 84 | 86 |
| Test group, % | 0 | 11 | 23 | 31 | 42 | 49 | 55 | 57 | 61 | 64 |

| Transdermal release test | | | | | | | | | | |
|---|---|---|---|---|---|---|---|---|---|---|
| Time, h | 0 | 4 | 8 | 12 | 16 | 20 | 24 | 28 | 32 | 36 |
| Control group, % | 0 | 21 | 38 | 43 | 49 | 53 | 55 | 56 | 56 | 58 |
| Test group, % | 0 | 27 | 50 | 67 | 72 | 80 | 84 | 92 | 96 | 96 |

| In vitro permeation test | | | | | | | | | | |
|---|---|---|---|---|---|---|---|---|---|---|
| Control group permeation depth | 239±56 µm | | | | Test group permeation depth (µm) | | | 550±18 µm | | |

### Comparative Example 1

A specific preparation method for a polypeptide-encapsulated quercetin molecular capsule was the same as that of Example 2, and merely changed a polypeptide encapsulation mode of the present disclosure to prepare the polypeptide-encapsulated quercetin molecular capsule. Specific operations were as follows.

According to a preparation method for a polypeptide-encapsulated quercetin molecular capsule, including following steps:
(1) D-β-alanine and L-histidine were uniformly mixed in a mass ratio of 1:1, distilled water in an amount twice a mass of the amino acid mixture was used to dissolve the amino acids, and ultrasonic enzymolysis was performed, using 5 wt% of a neutral protease (2,500 U/g, dry basis) based on the mass of the amino acids, at a temperature of 45°C, a pH value of 7, and an ultrasonic frequency of 150 W for 2.5 h to prepare an encapsulation precursor;
(2) the encapsulation precursor and the quercetin were uniformly mixed in a mass ratio of 4.5:1, and reaction was performed at a temperature of 45°C and a pH value of 7 under a nitrogen atmosphere for 2 h to prepare a molecular capsule precursor; and
(3) the molecular capsule precursor was added into a crystallizer, recrystallization of an outer structure was performed at a temperature of 33°C and a rotational speed of 350 rpm for 1.5 h, filtering was performed to retain a solid phase, then drying was performed at a temperature of 25°C and a pressure of 5 MPa for 4.5 h, and the polypeptide-encapsulated quercetin molecular capsule was prepared.

The molecular capsule prepared in this example was subjected to an in vitro release test, a transdermal release test, and an in vitro permeation test that are the same as those in Example 1.

Test results were shown in the following table:

| In vitro release test | | | | | | | | | | | |
|---|---|---|---|---|---|---|---|---|---|---|---|
| Time, h | 0 | 4 | 8 | 12 | | 16 | 20 | 24 | 28 | 32 | 36 |
| Control group, % | 0 | 23 | 32 | 45 | | 59 | 71 | 80 | 83 | 84 | 86 |
| Test group, % | 0 | 13 | 22 | 27 | | 31 | 41 | 47 | 51 | 53 | 58 |

| Transdermal release test | | | | | | | | | | | |
|---|---|---|---|---|---|---|---|---|---|---|---|
| Time, h | 0 | 4 | 8 | 12 | | 16 | 20 | 24 | 28 | 32 | 36 |
| Control group, % | 0 | 21 | 38 | 43 | | 49 | 53 | 55 | 56 | 56 | 58 |
| Test group, % | 0 | 22 | 41 | 58 | | 67 | 71 | 75 | 77 | 79 | 80 |

| In vitro permeation test | | | | | | | | | | | |
|---|---|---|---|---|---|---|---|---|---|---|---|
| Control group permeation depth | 239±56 µm | | | | Test group permeation depth (µm) | | | | 451±43 µm | | |

As can be clearly seen from the above table, in the present disclosure, the quercetin was directly encapsulated with the carnosine. Compared with the molecular capsule prepared in Example 2, it can be seen that the encapsulated quercetin exhibited a significant decrease in its in vitro release rate, along with fluctuations in release, failing to achieve effective and stable in vitro release. Furthermore, the transdermal release rate and the in vitro skin permeation depth were markedly reduced, leading to a significant decline in its practical efficacy and a decrease in the actual absorption rate of the quercetin.

### Comparative Example 2

A specific preparation method for a polypeptide-encapsulated quercetin molecular capsule was the same as that of Example 2, and merely changed a polypeptide encapsulation sequence of the present disclosure to prepare the polypeptide-encapsulated quercetin molecular capsule. Specific operations were as follows.

According to a preparation method for a polypeptide-encapsulated quercetin molecular capsule, including following steps:
(1) D-β-alanine, L-histidine, quercetin, and distilled water were uniformly mixed in a mass ratio of 1:1.5:0.6:4, and reaction was performed under a nitrogen atmosphere at a temperature of 45°C, a pH value of 7, and an ultrasonic frequency of 150 W for 4 h, during which 5 wt% of protease K (600 U/mL) based on a mass of the amino acids and 5 wt% of a neutral protease (2,500 U/g, dry basis) based on the mass of the amino acids were added, to prepare a molecular capsule precursor; and
(2) the molecular capsule precursor was added into a crystallizer, recrystallization of an outer structure was performed at a temperature of 33°C and a rotational speed of 350 rpm for 1.5 h, filtering was performed to retain a solid phase, then drying was performed at a temperature of 25°C and a pressure of 5 MPa for 4.5 h, and the polypeptide-encapsulated quercetin molecular capsule was prepared.

The molecular capsule prepared in the example was subjected to an in vitro release test, a transdermal release test, and an in vitro permeation test that are the same as those in Example 1.

Test results were shown in the following table:

| In vitro release test | | | | | | | | | | |
|---|---|---|---|---|---|---|---|---|---|---|
| Time, h | 0 | 4 | 8 | 12 | 16 | 20 | 24 | 28 | 32 | 36 |
| Control group, % | 0 | 23 | 32 | 45 | 59 | 71 | 80 | 83 | 84 | 86 |
| Test group, % | 0 | 12 | 23 | 27 | 30 | 39 | 47 | 51 | 55 | 56 |

| Transdermal release test | | | | | | | | | | |
|---|---|---|---|---|---|---|---|---|---|---|
| Time, h | 0 | 4 | 8 | 12 | 16 | 20 | 24 | 28 | 32 | 36 |
| Control group, % | 0 | 21 | 38 | 43 | 49 | 53 | 55 | 56 | 56 | 58 |
| Test group, % | 0 | 22 | 43 | 59 | 64 | 72 | 75 | 76 | 77 | 79 |

| In vitro permeation test | | | | | | | | | | |
|---|---|---|---|---|---|---|---|---|---|---|
| Control group permeation depth | 239±56 µm | | | | Test group permeation depth (µm) | | | 462±37 µm | | |

It can be clearly seen from the above table that achieving encapsulation of the quercectin concurrently with the construction of the carnosine yielded effects very similar to those of Comparative Example 1. Compared with the molecular capsule prepared in Example 2, it can be seen that the quercetin exhibited a decreased and fluctuated in vitro release rate, failing to achieve effective and stable in vitro release. Similarly, the transdermal release rate and the in vitro skin permeation depth were markedly reduced, leading to a significant decline in its practical efficacy and a decrease in the actual absorption rate of the quercetin.

### Comparative Example 3

A commercially available cyclodextrin-encapsulated quercetin nano capsule was subjected to an in vitro release test, a transdermal release test, and an in vitro permeation test that are the same as those in Example 1.

Test results were shown in the following table:

| In vitro release test | | | | | | | | | | |
|---|---|---|---|---|---|---|---|---|---|---|
| Time, h | 0 | 4 | 8 | 12 | 16 | 20 | 24 | 28 | 32 | 36 |
| Control group, % | 0 | 23 | 32 | 45 | 59 | 71 | 80 | 83 | 84 | 86 |
| Test group, % | 0 | 3 | 46 | 61 | 72 | 77 | 83 | 86 | 89 | 91 |

| Transdermal release test | | | | | | | | | | |
|---|---|---|---|---|---|---|---|---|---|---|
| Time, h | 0 | 4 | 8 | 12 | 16 | 20 | 24 | 28 | 32 | 36 |
| Control group, % | 0 | 21 | 38 | 43 | 49 | 53 | 55 | 56 | 56 | 58 |
| Test group, % | 0 | 13 | 22 | 31 | 42 | 47 | 47 | 49 | 51 | 52 |

| In vitro permeation test | | | | | | | | | | |
|---|---|---|---|---|---|---|---|---|---|---|
| Control group permeation depth | 239±56 µm | | | | Test group permeation depth (µm) | | | 337±63 µm | | |

It can be clearly seen from the above table that in vitro release of the cyclodextrin-encapsulated quercetin nano capsule had characteristics of super-efficient release between 4 h and 12 h.

However, the release rate during both the early and late stages was very gentle, and the nano capsule did not have characteristics of long-term, stable and sustained release, indicating that the encapsulation effect of the nano capsule was poor. In addition, in terms of the transdermal release rate, the nano capsule remained consistently low, even lower than that of the control group of the present disclosure, indicating that the cyclodextrin cannot effectively promote the transdermal absorption of the quercetin. Similarly, results of the in vitro permeation test further indicated that its permeation depth was far lower than that of the molecular capsule constructed by the technical solution of the present disclosure, which was also related to the differences between the encapsulating materials, i.e., the carnosine and the cyclodextrin.

### Application Example

The polypeptide-encapsulated quercetin molecular capsule prepared in Example 2 was used to prepare a moisturizing lotion, a repair eye cream, and a serum, and specific preparation modes were as follows.

Preparation of the moisturizing lotion:

| | Components | Ratio, % |
|---|---|---|
| Phase A | Deionized water | To 100 |
| | Carbomer | 0.25 |
| | EDTA disodium salt | 0.05 |
| | P-hydroxyacetophenone | 0.50 |
| | 1,2-hexanediol | 0.50 |
| | 1,3-butanediol | 3.00 |
| | Glycerinum | 7.00 |
| Phase B | PEG-100, stearate, glyceryl stearate | 1.50 |
| | Butyrospermum Parkii butter | 3.00 |
| | Cetostearyl alcohol | 1.00 |
| | Ethylhexyl palmitate | 2.50 |
| | Tocopheryl acetate | 0.50 |
| Phase C | 5% sodium hydroxide | 0.20 |
| Phase D | Ectoin | 0.5 |
| | Polypeptide-encapsulated quercetin molecular capsule | 0.11 |
| | Glycosylglycerol | 2 |

(1) Phase A was mixed and stirred evenly at a temperature of 80°C to 85°C;
(2) Phase B was heated, mixed, and dissolved at a temperature of 80°C to 85°C, then Phase B was added into Phase A, and stirring and homogenizing were performed for 5 min to 10 min; and
(3) Phase C was added into Phase A-B, stirring, uniform mixing, and homogenizing were performed for 5 min, then stirring was continued at a low speed to cool the mixture to a temperature of 40°C to 50°C, components of Phase D were added, and stirring and uniform mixing were performed to prepare the moisturizing lotion.

Preparation of the repair eye cream:

| | Components | Ratio, % |
|---|---|---|
| Phase A | Deionized water | To 100 |
| | Carbomer | 0.25 |
| | Xanthan gum | 0.20 |
| | EDTA disodium salt | 0.05 |
| | P-hydroxyacetophenone | 0.50 |
| | 1,2-hexanediol | 0.50 |
| | 1,3-butanediol | 3.00 |
| | Glycerinum | 7.00 |
| Phase B | Cetearyl glucoside | 3.00 |
| | Butyrospermum Parkii butter | 7.00 |
| | Cetostearyl alcohol | 4.00 |
| | Caprylic/capric triglyceride | 5.00 |
| | Tocopheryl acetate | 0.50 |
| Phase C | 5% sodium hydroxide | 0.20 |
| Phase D | Saccharomyces ferment lysate filtrate | |
| | 1,2-pentanediol | 5 |
| | 1,2-hexanediol | |
| | P-hydroxyacetophenone | |
| | Micrococcus lysate | |
| | Polypeptide-encapsulated quercetin molecular capsule | 0.5 |
| | Thiotaurine | 0.01 |

(1) Phase A was mixed and stirred evenly at a temperature of 80°C to 85°C;
(2) Phase B was heated, mixed, and dissolved at a temperature of 80°C to 85°C, then Phase B was added into Phase A, and stirring and homogenizing were performed for 5 min to 10 min; and
(3) Phase C was added into Phase A-B, stirring, uniform mixing, and homogenizing were performed for 5 min, then stirring was continued at a low speed to cool the mixture to a temperature of 40°C to 50°C, components of Phase D were added, and stirring and uniform mixing were performed to prepare the repair eye cream.

Preparation of the serum:

| | Components | Ratio, % |
|---|---|---|
| Phase A | Deionized water | To 100 |
| | Glycerinum | 4 |
| | 1,3-butanediol | 3 |
| | Carbomer | 0.3 |
| | P-hydroxyacetophenone | 0.6 |
| | 1,2-hexanediol | 0.6 |
| Phase B | Arginine | 0.3 |
| | Water | 1 |
| Phase C | Saccharomyces ferment lysate filtrate | |
| | 1,2-pentanediol | |
| | 1,2-hexanediol | 5 |
| | P-hydroxyacetophenone | |
| | Thermus thermophilus ferment | |
| | Erythrothioneine | 0.2 |
| | Polypeptide-encapsulated quercetin molecular capsule | 0.2 |

(1) raw material of Phase A were mixed at a temperature of 75°C to 85°C, and stirring and uniform mixing were performed until a uniform solution was formed;
(2) cooling was performed to a temperature of 40°C to 50°C, components of Phase B were added, and uniform mixing was performed; and
(3) stirring was performed at a low speed, components of Phase C were added one by one, and uniform stirring and mixing were performed to prepare the serum.

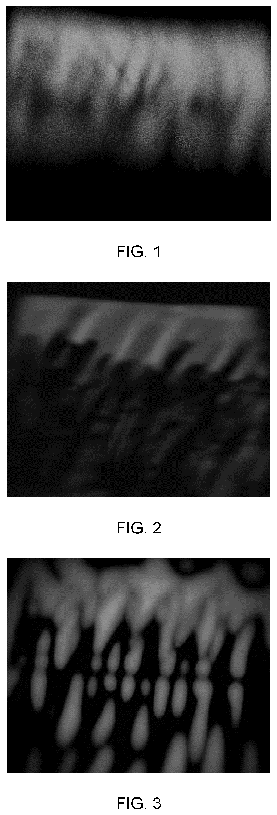

The in vitro permeation test used the abdominal skin of a three-month-old miniature pig. After depilation, the fat layer and part of the dermis were removed, the skin was cut into circular pieces with a diameter of 400 mm and fixed to a permeation device with the stratum corneum facing upward. FITC-HA was used as the fluorescein in the test. After the quercetin was crosslinked with the fluorescein, the drug was loaded by the encapsulation technology in this example, and then added into the above products. The products were diluted to a concentration of 50 µg/mL (based on the FITC-HA) through a PBS buffer solution (pH 7.4). 0.5 mL of the diluted solution was added to the permeation device and stored in the dark for 30 min. Subsequently, the skin was taken off and washed thoroughly with clean water until fluorescence on the surface layer was not discolored. The surface water was withdrawn with absorbent paper, the skin was placed in the dark for 10 min to slightly air-dry, and then the skin was sliced and placed under a fluorescence microscope for observation of fluorescence permeation in the skin. Characterization results were as shown in FIG. 1 to FIG. 3.

As can be seen from FIG. 1 to FIG. 3, the encapsulated quercetin had strong skin permeability, and the wider the fluorescent band, the deeper the drug permeation. Through comparison of the permeation abilities of the three cosmetics to the epidermis and dermis and the uptake abilities of cells in different skin layers for the quercetin, fluorescence microscopy revealed that the encapsulated quercetin can permeate the epidermis and reach the connective tissue of the dermis, such that dermal cells can be in full contact with the quercetin. Thus, it can be seen that encapsulation of quercetin by the technical method of the present disclosure can effectively improve the skin permeation of the quercetin.

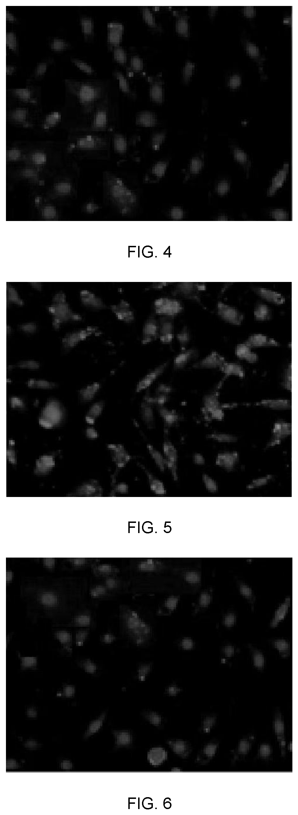

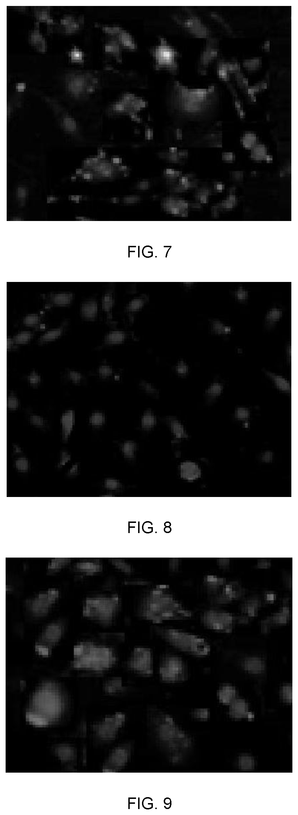

Based on this, a drug cellular uptake test was further performed: Mouse fibroblast cells (NCTC clone 929) were selected as the target cells, fluorescein isothiocyanate (isomer I) was selected as the intracellular fluorescence, and nuclear dye 4',6-Diamidino-2-phenylindole (DAPI) was selected for nuclear staining. After cross-linking of the quercetin and the fluorescein, the quercetin was encapsulated through the method of the above example to prepare the molecular capsule with a fluorescence effect. Then, the cells were treated with a fluorescent-labeled and encapsulated drug dissolved in the phosphate buffer solution. After fixation, permeation, and nuclear staining, an anti-fade mounting medium was added, and the drug uptake by cells after 4 h was photographed by a fluorescence microscope, and was compared with a control group. In the control group, the polypeptide-encapsulated quercetin molecular capsules were replaced by the same amount of a quercetin and L-carnosine composition. Characterization results were as shown in FIG. 4 to FIG. 9. With reference to FIG. 4 to 9, the blue fluorescence represented the cell nuclei, and the green fluorescence represented the drug uptake by cells. The higher the green fluorescence intensity, the better the uptake. Because it is difficult to develop color in the figures, the drug uptake can be roughly estimated according to the size of the light spots. When the drug was taken up, the larger the light spot, the greater intracellular accumulation of the drug, and thus better cellular uptake capacity for the drug. As can be seen from the figures, compared with the control group, the uptake rate of the cells for the polypeptide-encapsulated quercetin molecular capsule of the present disclosure was much higher than that for a quercetin stock solution. Thus, it is evident that the polypeptide-encapsulated quercetin is significantly improved in an intracellular uptake ability of the quercetin.

## Claims

1. A preparation method for a polypeptide-encapsulated quercetin molecular capsule, comprising steps of:
(1) mixing amino acid A, quercetin, and water in a ratio, followed by ultrasonic enzymolysis to prepare a precursor;
(2) mixing the precursor with amino acid B in a ratio to prepare a molecular capsule precursor; and
(3) adding the molecular capsule precursor into a crystallizer for recrystallization of an outer structure, and performing filtering and high-pressure drying to prepare the polypeptide-encapsulated quercetin molecular capsule.

2. The preparation method for a polypeptide-encapsulated quercetin molecular capsule according to claim 1, wherein
the amino acid A in the step (1) is L-histidine; and
the amino acid A, the quercetin, and the water are mixed in a mass ratio of (0.9 to 1.2):1:(3 to 5) in the step (1).

3. The preparation method for a polypeptide-encapsulated quercetin molecular capsule according to claim 1, wherein
the ultrasonic enzymolysis in the step (1) is performed using a neutral protease having an activity of 2,000 U/g to 3,000 U/g on a dry basis; and
the neutral protease is used in an amount of 8 wt% to 12 wt% based on the quercetin.

4. The preparation method for a polypeptide-encapsulated quercetin molecular capsule according to claim 1, wherein
the amino acid B in the step (2) is a mixture of D-β-alanine and L-histidine; and
in a total amount of the amino acid B, a content of the L-histidine ranges from 28 wt% to 32 wt%, with the balance being the D-β-alanine.

5. The preparation method for a polypeptide-encapsulated quercetin molecular capsule according to claim 1 or 4, wherein
during the preparation of a molecular capsule precursor in the step (2), a reaction temperature is controlled at a range from 45°C to 50°C, a pH value of a reaction system is adjusted at a range from 6.8 to 7.2, and in a reaction process, liquid proteinase K having an activity of 600 U/mL is added in an amount from 4 wt% to 6 wt% based on the amino acid B.

6. The preparation method for a polypeptide-encapsulated quercetin molecular capsule according to claim 1, wherein
the recrystallization in the step (3) is performed at a temperature of 30°C to 35°C, a rotational speed of the crystallizer is controlled at a range from 300 rpm to 400 rpm during the recrystallization, and the recrystallization lasts for 1 h to 2 h.

7. The preparation method for a polypeptide-encapsulated quercetin molecular capsule according to claim 1, wherein
the high-pressure drying is performed at a temperature of 24°C to 26°C and a pressure of 4 MPa to 6 MPa for 4 h to 5 h.

8. A polypeptide-encapsulated quercetin molecular capsule, prepared by the method according to any one of claims 1 to 7.
